(19) Europäisches Patentamt
European Patent Office
Office européen des brevets

(11) Publication number: **0 510 250 A2**

# (12) EUROPEAN PATENT APPLICATION

(21) Application number: **91115983.8**

(51) Int. Cl.[5]: **C07C 247/04**

(22) Date of filing: **19.09.91**

(30) Priority: **22.04.91 US 697354**

(43) Date of publication of application:
**28.10.92 Bulletin 92/44**

(84) Designated Contracting States:
**CH DE FR GB IT LI**

(71) Applicant: **ROCKWELL INTERNATIONAL CORPORATION**
**2230 East Imperial Highway**
**El Segundo California 90245(US)**

(72) Inventor: **Wilson, Edgar Roland**
**7888 Lilac Lane**
**SIMI VALLEY, CALIFORNIA 93063(US)**
Inventor: **Weber, James Frederic**
**4376 Brookglen Street**
**MOORPARK, CALIFORNIA 93021(US)**

(74) Representative: **Wächtershäuser, Günter, Dr.**
**Tal 29**
**W-8000 München 2(DE)**

(54) **Azide-terminated azido compound.**

(57) Glycidal azide polymer azide prepared by reacting in a refluxing azueous solution, polyepichlorohydrin with sodium azide and a phase transfer catalyst such as dimethyl-dicoco-ammonium chloride.

EP 0 510 250 A2

Background of the Invention

1. Field of the Invention

This invention relates to an energetic azide-terminated azido plasticizer, and is particularly directed to a method for producing this compound.

2. Description of Related Art

U.S. Patent 4,781,861 discloses a process for preparing a glycidal azide polymer azide having the following structural formula:

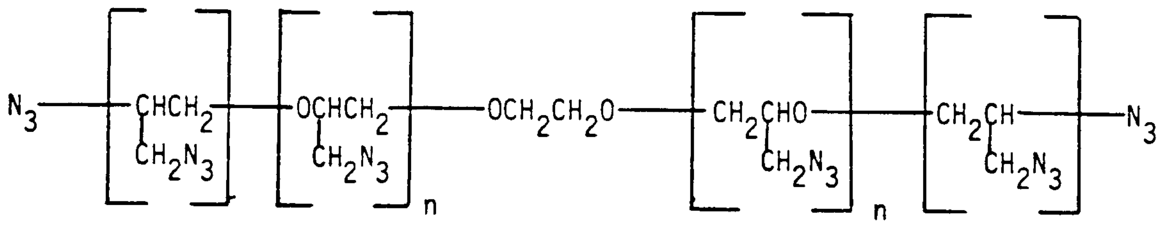

in which n is an integer from 0 to 9 and said glycidal azide polymer azide is produced by the process of reacting polyepichlorohydrin-nitrate of the general formula:

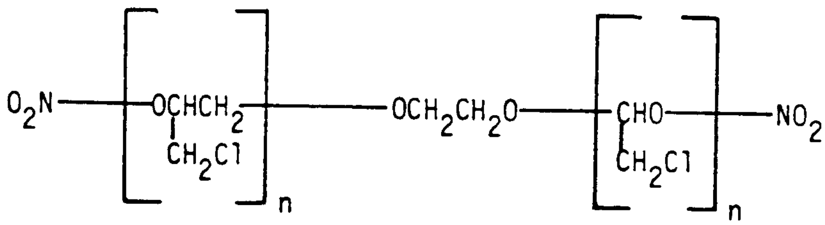

in which n is an integer from 1 to 10, with sodium azide in a polar solvent.

Summary of the Invention

The present invention is for a process whereby glycidal azide polymer azide is produced by means of an aqueous phase transfer procedure.

Objects of the Invention

Therefore, it is an object of the present invention to provide materials useful in formulating solid propellants, explosives, and the like.

Another object of the present invention is to provide a new process for producing glycidal azide polymer azide.

Other objects of the present invention will become apparent from a reading of the description of the preferred embodiment and claims.

Description of the Preferred Embodiment

In accordance with the present invention there is provided a glycidal azide polymer azide having the following general formula:

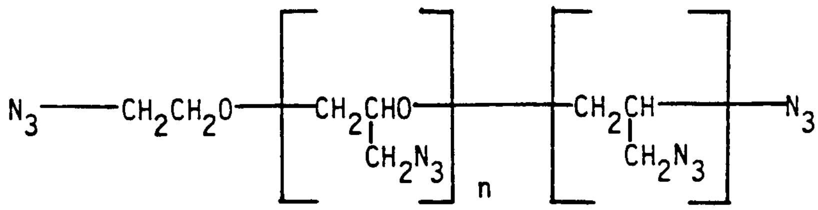

where n is an integer from about 4 to about 9, prepared by reacting in a refluxing aqueous solution, polyepichlorohydrin having the following general formula:

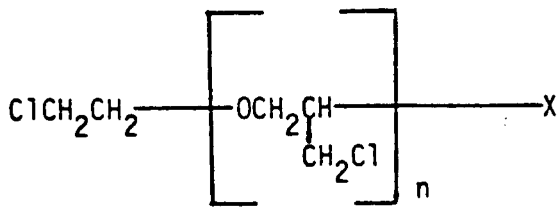

where n is an integer from about 5 to about 10 and X is a leaving group such as chloride, bromide, or a sulfonate ester including, but not limited to methane sulfonate, benzene sulfonate, or toluene sulfonate with sodium azide and a phase transfer catalyst of the general formula;

$R^1R^2R^3R^4N^+Z^-$

where the R groups are alkyl groups having from 1 to about 10 carbon atoms and Z is an anion such as chloride, bisulfate, carbonate, or bromide. A preferred catalyst is dimethyl-dicoco-ammonium chloride in which two R groups are $CH_3$, and two of the R groups are C-8 to C-10.

By way of example and without limitation, the method of producing glycidal azide polymer azide according to the present invention is set forth in the example.

EXAMPLE

In a suitable reaction vessel, a solution of 100.0 grams (1.02 moles) polyepichlorohydrin was reacted with 79.6 grams (1.22 moles) of sodium azide in a refluxing aqueous solution containing 20.0 grams (20 weight percent) of the phase transfer catalyst dimethyl-dicoco-ammonium chloride.

This reaction mixture was stirred for 88 hours at about 106° C. The organic solution was next washed with 200 milliliters of water 2 times, 100 milliliters of 50% aqueous isopropyl alcohol 4 times, and subsequently dried over anhydrous magnesium sulfate followed by passage through a silica gel column. Concentration of the final solution gave 63.7 grams (75%) of glycidal azide polymer azide.

In the reaction of sodium azide with polyepichlorohydrin (PECH) in the polar solvent dimethylsulfoxide (DMSO) to produce glycidyl azide polymers, undesirable side reactions between PECH and DMSO and between DMSO and sodium azide may occur. These side reactions can adversely affect the quality of the product by introducing impurities. If water is used as the solvent in place of DMSO, the reaction is immeasurably slow because the sodium azide is soluble in water. Thus, the two reactants are unable to fully interact.

A phase transfer catalyst, viz., dimethyl-dicoco-ammonium chloride, acts to carry the azide ion from the aqueous phase to the (non-aqueous) PECH phase where it can react with the PECH to form the desired product.

The mechanism of this transfer is that the quaternary ammonium cation of the phase transfer agent exchanges with the azide ion of sodium azide to form an organic soluble quaternary ammonium azide. In return, as the PECH reacts with the azide, the resultant chloride ion is carried by the ammonium ion back to the aqueous phase to be exchanged again for another azide ion. The result is a reaction in an aqueous medium at a practical rate which gives a higher quality product than achieved in the presence of DMSO.

The product prepared according to the present invention has been characterized and identified as set

forth in the attached Table 1.

TABLE

| | | | |
|---|---|---|---|
| NAME: | GLYCIDYL AZIDE POLYMER AZIDE | | |
| STRUCTURE: | $N_3CH_2CH_2-[OCH_2CH(CH_2N_3)]_5-N_3$ | | |
| FORMULA: | $C_{17}H_{29}N_{21}O_5$ | | |
| MOLECULAR WEIGHT: | 607 | | |
| ELEMENTAL ANALYSES: | C | H | N |
| Calculated: | 33.60 | 4.78 | 48.43 |
| Found: | 34.03 | 4.87 | 47.00 |
| INFRARED SPECTRUM: | $N_3$(4.7μ;2145 $cm^{-1}$) | | |
| APPEARANCE: | Light Yellow Liquid | | |
| REFRACTIVE INDEX: | 1.5143 at 25°C | | |
| DENSITY: | 1.27 g/cc at 25°C | | |
| FREEZING POINT: | <56°C | | |
| DSC: | 224°C | | |
| WEIGHT LOSS: | 0.48% after 48 hrs at 74°C | | |
| IMPACT SENSITIVITY: | 40 in-lb | | |
| ELECTROSTATEIC SENSITIVITY: | > 18 Joules | | |
| FRICTION SENSITIVITY: | < 36 Kg (BAM tester) | | |
| $\Delta H_f$ (estimated) | + 337 Kcal/mole | | |

Obviously, variations and modification may be made without departing from the present invention. Accordingly, it should be clearly understood that the form of the present invention described above is illustrative only and is not intended to limit the scope of the present invention.

## Claims

**1.** A process of preparing a glycidal azide polymer azide having the following general formula:

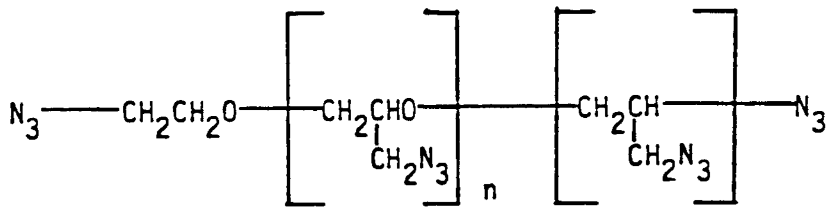

where n is from 4 to 9, by reacting in a refluxing aqueous solution, polyepichlorohydrin of the general formula:

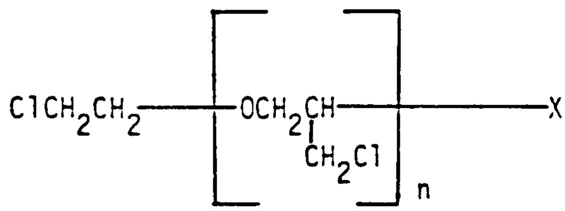

where n is from 5 to 10 and X is a chloride, bromide or sulfonate ester, with sodium azide and a phase transfer catalyst of the general formula:

$R^1R^2R^3R^4N^+Z^-$,

where the R groups are alkyl groups having from 1 to about 10 carbon atoms and Z is an anion selected from the group consisting of chloride, bisulfate, carbonate, or bromide.

**2.** The process of claim 1 wherein the phase transfer catalyst is dimethyl-dicoco-ammonium chloride.